# EUROPÄISCHE PATENTSCHRIFT

(11) **EP 1 374 785 B1**
(45) Veröffentlichungstag und Bekanntmachung des Hinweises auf die Patenterteilung: **21.05.2008**
(21) Anmeldenummer: 03014395.2
(22) Anmeldetag: 26.06.2003
(51) Int. Cl.: A61B 17/22, A61B 8/08

(54) **Lithotripter mit einer Doppler-Ultraschalleinheit zur Trefferüberwachung**
Lithotripter with a doppler ultrasound unit for hit/miss monitoring
Lithotripteur avec une unité de doppler à ultrason pour le contrôle d'impacts

(30) Priorität: 26.06.2002 DE 10228550
(43) Veröffentlichungstag der Anmeldung: 02.01.2004
(73) Patentinhaber: Dornier MedTech Systems GmbH, 82234 Wessling (DE)
(72) Erfinder: Bohris, Christian, Dr., 82234 Wessling (DE); Bayer, Thomas, Dr., 87493 Lauben (DE)
(74) Vertreter: Grünecker, Kinkeldey, Stockmair & Schwanhäusser Anwaltssozietät

(56) Entgegenhaltungen:
- DE-A- 4 012 760
- DE-A- 4 446 192
- HÖRBARTH K, HOFBAUER J, MARBERGER M: "Color flow Doppler sonography for extracorporal shock wave lithotripsy" JOURNAL OF UROLOGY, Bd. 150, Nr. 6, 1993, Seiten 1768-1770, XP009019789

## Beschreibung

Die vorliegende Erfindung betrifft einen Lithotripter zur Fragmentierung eines Zielobjekts, insbesondere eines Konkrements, in einem vorzugsweise menschlichen Körper, umfassend einen Stoßwellengenerator zur Erzeugung fokussierter Stoßwellen, eine Ultraschall-Sende/Empfangseinheit mit einem Ultraschall-Transducer zum Aussenden von Ultraschallwellen in den Körper und zum Empfangen von in einem Zielgebiet des Stoßwellengenerators reflektierten Ultraschallwellen, und eine an die Ultraschall-Sende/Empfangseinheit angeschlossene Dopplersignaleinheit zum Generieren und Auswerten eines Dopplersignals aus den ausgesandten und empfangenen Ultraschallwellen.

Lithotripter sind heutzutage als medizinische Geräte zur Zertrümmerung von Konkrementen, beispielsweise Nierensteinen, im Körper eines Patienten mit Hilfe fokussierter Stoßwellen weitverbreitet. Derartige Lithotripter werden von der Anmelderin gewerblich angeboten, beispielsweise unter der Bezeichnung "Dornier Lithotripter S" oder "Dornier Compact Delta". Bei all diesen Geräten muß das Konkrement vor Beginn der Behandlung geortet werden, damit der Patient mit Hilfe einer verschiebbaren Liege so positioniert werden kann, daß sich beispielsweise sein Nierenstein im Fokus der Stoßwellen befindet, die mit Hilfe des Stoßwellengenerators des Lithotripters erzeugt werden. Diese anfängliche "Justage", d.h. Positionierung des Patienten erfolgt üblicherweise mit einer bildgebenden Ortungsvorrichtung, etwa einem bildgebenden Ultraschall-Scanner oder einer Röntgenortungsvorrichtung. Diese dient zusätzlich zur anfänglichen erstmaligen Ortung des Konkrements vor Beginn der ESWL-Behandlung (extrakorporale Stoßwellenlithotripsie) oder der ESWT-Behandlung (extrakorporale Stoßwellentherapie) auch dazu, während des Verlaufs der Behandlung die Position des Konkrements kontinuierlich zu überwachen, um sicherzustellen, daß es nicht im Körper des Patienten verrutscht bzw. an einen anderen Ort gewandert ist oder daß sich der Patient nicht auf seiner Liege bewegt hat, so daß sich das Konkrement möglicherweise nicht mehr im Fokus der Stoßwellen befindet. Für einen umfassenden Überblick über technische und medizinische Aspekte der ESWT und der bei Lithotriptern eingesetzten Geräte wird auf das Buch "ESWT and Ultrasound Imaging of the Musculosceletal System", Steinkopff-Verlag, Darmstadt, 2001, ISBN 3-7985-1252-3 verwiesen.

Üblicherweise wird die genannte bildgebende Ortungsvorrichtung auch dazu verwendet, während des Verlaufs der Behandlung den Erfolg der Lithotripsie zu überwachen, d.h. die Fragmentierung des Zielobjekts. Da eine Behandlung typischerweise ca. 30 Minuten dauert, kann wegen der zu hohen Strahlenbelastung nicht kontinuierlich geröntgt werden, sondern allenfalls in Intervallen von 3 bis 5 Minuten. Verlagert sich in der Zwischenzeit das Zielobjekt zum Beispiel durch eine Bewegung des Patienten, so wird bis zur nächsten Kontrollaufnahme der Körper durch Stoßwellen belastet, ohne daß das Zielobjekt weiter fragmentiert wird, da es sich nicht mehr im Stoßwellenfokus befindet.

Bei Lithotriptern, bei denen als bildgebende Ortungsvorrichtung ein Ultraschallscanner eingesetzt wird, kann dieser zwar kontinuierlich zur Visualisierung des Zielobjekts eingesetzt werden, allerdings ist die Ortung u.a.wegen der Bildqualität oft wesentlich schwieriger als bei Röntgenaufnahmen, so daß selbst erfahrenes medizinisches Personal oft Schwierigkeiten hat, das Zielobjekt zu erkennen oder gar seinen Fragmentierungsgrad einzuschätzen.

Darüberhinaus hat die Verwendung derartiger bildgebender Ortungsvorrichtungen nicht nur zur "Anfangsjustage" des Patienten, sondern auch zur "Trefferkontrolle" im Verlauf der Behandlung folgenden grundsätzlichen Nachteil: Zwar läßt sich hierdurch die Position des Zielobjekts relativ zum Stoßwellenfokus kontrollieren, d.h. eine rein geometrische Größe; es läßt sich jedoch nicht die Wirkung der Stoßwellen auf das Zielobjekt selbst feststellen. Grundsätzliche Probleme wie eine mangelnde Ankopplung des Stoßwellengeräts an den Körper des Patienten, eine Abschattung von Stoßwellen, beispielsweise durch Rippen etc., werden daher unter Umständen erst spät erkannt, wenn nämlich im Therapieverlauf keinerlei Effekte am Zielobjekt sichtbar werden.

Daher wurden im Stand der Technik bereits Ultraschall-Dopplerverfahren zur kontinuierlichen Trefferkontrolle vorgeschlagen. Es wurde nämlich angenommen, daß das Konkrement im menschlichen Körper bei einem Treffer aufgrund des Impulsübertrags von der Stoßwelle eine makroskopische Bewegung ausführt. Bestrahlt man das Zielobjekt mit Ultraschallwellen und mißt die an ihm reflektierten Ultraschallwellen, so äußert sich diese makroskopische Bewegung in einer Dopplerverschiebung der Frequenz der reflektierten Wellen. Dementsprechend ausgestattete Lithotripter gemäß dem Oberbegriff des Anspruchs 1 sind beispielsweise aus der EP 0 367 116 B1, der EP 0 548 048 B1 und der DE 44 46 192 A1 bekannt. Diesen Geräten ist gemeinsam, daß der Ultraschall-Transducer Ultraschallwellen in den Körper aussendet, die vom Körper zum Ultraschall-Transducer zurückreflektierten Ultraschallwellen erfaßt, wobei eine Dopplersignaleinheit aus den ausgesandten und empfangenen Ultraschallwellen ein Dopplersignal generiert und auswertet, wobei im wesentlichen der Betrag einer Frequenzverschiebung des reflektierten Signals gegenüber den ausgesandten Wellen berechnet wird und hieraus auf die Treffgenauigkeit geschlossen wird.

Diese Vorgehensweise weist verschiedene Nachteile auf:

Es ist im Stand der Technik bereits bekannt, daß das Dopplersignal gerade in der Nähe seines zeitlichen Nullpunkts, d.h. unmittelbar nach dem Aussenden der Stoßwelle, Artefakte enthält. Um zu verhindern, daß genau solche Artefakte gemessen werden, weisen beispielsweise die Lithotripter gemäß der EP 0 367 116 B1 und der EP 0 548 048 B1 Mittel zur zeitlichen Synchronisierung zwischen dem Stoßwellengenerator und der Dopplersignaleinheit auf, was diese Geräte aufwendig und teuer macht.

Darüber hinaus kann es in der Umgebung von starken Streuern wie einem Konkrement im Dopplerspektrum zu dem sogenannten Spiegelartefakt kommen. Durch Zweifachreflektion wird die Bewegung eines Streuers zusätzlich in entgegengesetzer Richtung registriert. Dies führt zu einem zusätzlichen Betrag, der dem an der Nullinie gespiegelten Nutzsignal entspricht. Wegen der Kurzlebigkeit des Prozesses und der starken Artefakte können den Spektren nicht einfach Geschwindigkeits-ZeitVerläufe und somit eine Trefferinformation ugeordnet werden.

Es ist daher Aufgabe der vorliegenden Erfindung, enen Lithotripter der eingangs genannten Art bereitzustellen, der aus dem Dopplersignal eine Information im Hinblick auf die Treffer- und Desintegrationskontrolle ermittelt und zur Darstellung bringt und keine aufwendigen Mittel zur Synchronisierung des Stoßwellengenerators mit der Dopplersignaleinheit erfordert.

Insbesondere wird die oben genannte Aufgabe bei einem erfindungsgemäßen Lithotripter dadurch gelöst, daß die Dopplersignaleinheit dazu ausgelegt ist, die Zeitdauer des Dopplersignals zu bestimmen und ein zugeordnetes Zeitdauersignal anzugeben.

Die Zeitdauer des Dopplersignals ist weitgehend unabhängig von den oben genannten Artefakten und ein charakteristisches Maß für einen Treffer bzw. Fehlschuß. Darüber hinaus läßt sich durch die Anzeige dieses Parameters über die Behandlung auch die Desintegrationswirkung bestimmen.

Die erfindungsgemäße Ausgestaltung der Dopplersignaleinheit basiert auf neuen in-vitro- und klinischen Experimenten, bei denen jeweils der gesamte zeitliche Verlauf des Dopplersignals, d.h. die im reflektierten Signal auftretende Frequenzverschiebung gegenüber dem ausgesandten Signal als Funktion der Zeit untersucht wurde. Bei den in-vitro-Experimenten wurde die Frage, ob und mit welcher Genauigkeit eine Stoßwelle das Zielobjekt getroffen hat, mit Hochgeschwindigkeitsfotografien überwacht. Als wichtigstes Ergebnis wurde übereinstimmend festgestellt, daß die Zeitdauer des Dopplersignals ein charakteristisches Maß für einen "Treffer" darstellt. Anders ausgedrückt kann dem zugeordneten Zeitdauersignal, das von der erfindungsgemäßen Dopplersignaleinheit ausgegeben wird, entnommen werden, ob und in welchem Maße eine Stoßwelle das Zielobjekt getroffen hat. Grundsätzlich gilt hierbei, daß die Zeitdauer des Dopplersignals bei stärkerer Wirkung der Stoßwelle auf das Zielobjekt zunimmt. Befindet sich das Zielobjekt hingegen außerhalb des Stoßwellenfokus, so ist die Zeitdauer des Dopplersignals signifikant kürzer als bei einem Treffer. Zerfällt das Zielobjekt, beispielsweise ein Konkrement, im Laufe der Behandlung in eine zunehmende Zahl von kleineren Fragmenten, so nimmt die Zeitdauer des Dopplersignals zu. Durch die Zunahme der Signallänge im Verlauf der Behandlung kann die Desintegrationswirkung der Schockwellentherapie abgelesen werden.

An dieser Stelle ist auf die zugrundeliegenden Wechselwirkungsmechanismen zwischen der Stoßwelle und dem Zielobjekt einzugehen: Im Stand der Technik wurde bislang davon ausgegangen, daß es nur einen einzigen Wechselwirkungsmechanismus gibt, nämliche eine makroskopische Bewegung des Konkrements, wenn es erfolgreich von der Stoßwelle getroffen wird. Die genannten neueren Untersuchungen zeigen jedoch, daß dieser Mechanismus nur einer von mehreren Mechanismen ist: Man beobachtet zwar tatsächlich solche makroskopische Bewegungen des Konkrements, insbesondere dann, wenn es bereits teilweise oder sogar größtenteils fragmentiert ist. Gerade am Anfang der Behandlung, wenn das Konkrement noch weitgehend unfragmentiert ist, dominieren jedoch andere Effekte, nämlich insbesondere das Herausschießen von Fragmenten aus dem Konkrement sowie das Auftreten von Kavitationsblasen um das Konkrement herum bei einem erfolgreichen Treffer. All diese Mechanismen führen zur Dopplerverschiebung bei den. Frequenzen der reflektierten Schallwellen und somit zum beobachteten Dopplersignal und führen dazu, daß dessen Zeitdauer um so größer ist, je stärker die Wechselwirkung zwischen Stoßwelle und Stein ist und je stärker das Konkrement bereits fragmentiert ist.

Hinsichtlich der Weiterverarbeitung der im Zeitdauersignal enthaltenen Trefferinformation sind zahlreiche Vorgehensweisen möglich:

In einer bevorzugten Weiterbildung des erfindungsgemäßen Lithotripters ist vorgesehen, daß er ferner eine mit der Dopplersignaleinheit verbundene Anzeigevorrichtung zur Anzeige von Dopplersignalen und/oder eine Alarmvorrichtung aufweist, der das Zeitdauersignal zugeführt wird. Die momentanen Dopplersignale werden, wie oben bereits erwähnt, in einem Koordinatensystem dargestellt, dessen Abszisse die Zeit und deren Koordinate die per Fourier-Transformation aus den reflektierten Ultraschallwellenamplituden berechnete Frequenzverschiebung angibt. Insbesondere kann im Verlauf der gesamten Behandlung die gemessene Zeitdauer der Dopplersignale in Form einer fortlaufenden Anzeige dargestellt werden, um die Entwicklung der Dopplersignallänge zu verfolgen. Mit erfolgreicher fortschreitender Zertrümmerung des Zielobjekts sollte die Dopplersignallänge kontinuierlich zunehmen. Nimmt die Signallänge jedoch ab, so ist dies ein starkes Indiz dafür, daß sich das Konkrement aus dem Stoßwellenfokus herausbewegt hat, sei es durch eine Konkrementbewegung (beispielsweise die Bewegung eines Nierensteins innerhalb der Niere des Patienten) oder durch eine Verschiebung des Patienten selbst. Als Anzeigevorrichtung kann hierbei gegebenenfalls der Bildschirm verwendet werden, auf dem auch die von der bildgebenden Ortungsvorrichtung gelieferten Bilder dargestellt werden. Die erfindungsgemäß vorgeschlagene Alarmvorrichtung kann akustisch und/oder optisch arbeiten und beispielsweise einen Warnton aussenden oder eine Warnleuchte einschalten, sobald sich aus dem zugeführten Zeitdauersignal ergibt, daß die Zeitdauer des Dopplersignals unter einen vorbestimmten Wert gefallen ist, was auf einen "Fehlschuß" hinweist. Zusätzlich oder alternativ dazu kann der Alarm dann ausgelöst werden, wenn das Zeitdauersignal einen negativen Trend aufweist. Bei einer Desintegration ist nämlich von einer steigenden Signallänge auszugehen.

Alternativ oder zusätzlich kann vorgesehen sein, daß der Stoßwellengenerator mit der Dopplersignaleinheit verbunden und dazu ausgelegt ist, die Erzeugung von Stoßwellen als Funktion des Zeitdauersignals zu stoppen bzw. fortzusetzen. Hierdurch läßt sich eine automatische Abschaltung des Stoßwellengenerators erreichen, wenn offenbar das Zielobjekt nicht mehr im Stoßwellenfokus liegt. Somit wird unabhängig von der Aufmerksamkeit oder der Reaktionszeit des den Lithotripter bedienenden medizinischen Personals eine unnötige Belastung des Körpers des Patienten durch Stoßwellen-Fehlschüsse vermieden.

Zweckmäßigerweise ist der Ultraschall-Transducer der Ultraschall-Sende/Empfangseinheit an einem einstellbaren Halter montiert. Der Ultraschall-Transducer kann dann unabhängig von anderen Teilen des Lithotripters zur Optimierung des Dopplersignals justiert und in der optimalen Position festgestellt werden. Insbesondere kann hierdurch gewährleistet werden, daß der Ultraschall-Transducer auf den Stoßwellenfokus gerichtet ist. Alternativ zu dieser sogenannten isozentrischen Scannerführung kann auch ein sogenannter Inline-Transducer eingesetzt werden, d.h. ein Ultraschall-Transducer, der in die Stoßwellenquelle integriert ist.

Grundsätzlich ist hierbei folgendes zu beachten: Durch den einstellbaren Halter wird lediglich eine Linie festgelegt, entlang der sich beispielsweise bei einem PW-Verfahren (pulse wave) ein ausgesandter Ultraschallpuls im Gewebe ausbreitet und Echos generiert werden. Bei der isozentrischen Bauweise ist dann gewährleistet, daß sich der Stoßwellenfokus, d.h. das Zielgebiet, auf dieser Linie befindet. Durch bekannte Mittel, beispielsweise einen Weggeber, läßt sich der Abstand des Transducers zum Fokus bestimmen. Aufgrund der bekannten Laufzeit von Ultraschallpulsen im Gewebe läßt sich aus diesem Abstand wiederum ein Zeitfenster definieren, das denjenigen Anteil des Echos herausschneidet, der im Zielgebiet generiert wurde. Alternativ zu diesem Puls-Echo-Verfahren kann ein Dopplersignal auch durch einen CW-Transducer (continuous wave) generiert werden, bei dem ein Sendeelement kontinuierlich Ultraschallwellen aussendet und ein Empfangselement kontinuierlich das Echo empfängt. Bei dieser einfacheren Ausführung ist jedoch keine Tiefenselektion möglich.

Die Ermittlung der wesentlichen Information des Dopplersignals, nämlich ihrer Zeitdauer, in der Dopplersignaleinheit kann auf verschiedene Weisen erfolgen: Beispielsweise kann die Dopplersignaleinheit dazu ausgelegt sein, als Zeitdauer des Dopplersignals ein Zeitintervall zu bestimmen, innerhalb dessen der Betrag des Dopplersignals auf einen vorbestimmten Bruchteil eines Anfangsbetrags gefallen ist, insbesondere auf den e-ten Teil des Anfangsbetrags. Eine derartige Auslegung der Dopplersignaleinheit ist dann vorteilhaft, wenn der Betrag eines Dopplersignals jeweils weitestgehend einer exponentiell abfallenden Einhüllenden-Kurve entspricht, da dann die Zeitdauer des Dopplersignals durch automatisiertes Anpassen von Test-Exponentialfunktionen (Fitten) schnell und zuverlässig ermittelt werden kann.

Wenn hingegen der zeitliche Verlauf des Betrags eines Dopplersignals von einem exponentiellen Abfall der Einhüllenden-Kurve abweicht, so empfiehlt es sich in einer besonders vielseitigen Ausführungsform des erfindungsgemäßen Lithotripters, daß die Dopplersignaleinheit dazu ausgelegt ist, als Zeitdauer des Dopplersignals ein Zeitintervall zwischen einem Signal-Anfangszeitpunkt und einem Signal-Endzeitpunkt zu bestimmen, wobei der Signal-Anfangszeitpunkt festgestellt wird, wenn der Betrag des Dopplersignals einen ersten Schwellenwert überschreitet, und der Signal-Endzeitpunkt festgestellt wird, wenn der Betrag des Dopplersignals einen zweiten Schwellenwert unterschreitet, wobei in einer einfachen Weiterbildung dieser Ausführungsform der erste und der zweite Schwellenwert identisch sein können.

Grundsätzlich können diese Schwellenwerte in der Dopplersignaleinheit des erfindungsgemäßen Lithotripters voreingestellt sein und hierbei sowohl die technischen Daten des Stoßwellengenerators (insbesondere seine Leistung) als auch typische Patientendaten berücksichtigen. Zweckmäßigerweise ist jedoch vorgesehen, daß die Dopplersignaleinheit Einstellmittel zum Einstellen des ersten und/oder des zweiten Schwellenwerts aufweist. Dann können diese Schwellenwerte zu Beginn der Behandlung abhängig vom auftretenden Rauschen des Dopplersignal-Untergrunds individuell eingestellt werden. Die Schwellenwerte können jedoch auch automatisch aus dem Signal selbst bestimmt werden. Aus den vor dem Signal-Anfangszeitpunkt akquirierten Signalen läßt sich nämlich ein Grundrauschen bestimmen. Die Schwellenwerte können dann als ein vorbestimmtes Vielfaches der Rauschamplitude definiert sein und von der erfindungsgemäßen Dopplersignaleinheit automatisch eingestellt werden.

In einer vorteilhaften Weiterbildung des erfindungsgemäßen Lithotripters kann die Dopplersignaleinheit ferner dazu ausgelegt sein, die Zeitdauer des Dopplersignals über mehrere Stoßwellen zu mitteln. Hierfür sind die folgenden geometrischen Umstände einer typischen Lithotripsie-Behandlung zu berücksichtigen: Der Stoßwellenfokus hat typischerweise eine Ausdehnung von ca. 4 mm. Ein typisches Konkrement, beispielsweise ein Nierenstein, weist am Anfang der Behandlung Abmessungen zwischen 5 und 20 mm auf, und seine Hin- und Herverlagerung durch die reine Atmung des Patienten kann mit einer Amplitude von ca. 30 mm erfolgen. Unter diesen Bedingungen würden, da das Aussenden von Stoßwellen typischerweise ohne Berücksichtigung des jeweiligen Atmungszustands des Patienten erfolgt, zwangsläufig einige Stoßwellen das Konkrement verfehlen. Die im unmittelbaren Anschluß an derartige Stoßwellen gemessenen Dopplersignal-Zeitdauem sind - wie oben bereits erläutert - besonders kurz, so daß je nach Verwendung des zugeordneten Zeitdauersignals beispielsweise die Alarmvorrichtung betätigt oder der Stoßwellengenerator abgeschaltet würde, obwohl keinerlei Dejustage gegeben ist. Vielmehr würden bereits die nächsten Stoßwellen wieder das Konkrement treffen, da sie bei einem anderen Atmungszustand des Patienten ausgesandt werden. Bei der in dieser erfindungsgemäßen Ausführungsform vorgesehenen Mittelung der Zeitdauer des Dopplersignals über mehrere Stoßwellen kann zunächst in einer Ersteinstellung der Dopplersignaleinheit eine Mittelung über beispielsweise fünf Stoßwellen erfolgen, es empfiehlt sich jedoch zur weiteren Steigerung der Behandlungseffizienz, die Zahl der Dopplersignale, über die gemittelt wird, zu Beginn der Behandlung des Patienten abhängig von seinem typischen Atmungsverhalten, von der Größe des zu fragmentierenden Konkrements etc. individuell einzustellen. Grundsätzlich sind anstatt einer Mittelung auch andere Arten der Signalfilterung möglich, z.B. eine Median-Filterung.

Nicht nur hinsichtlich der Zahl der Dopplersignale, über die zweckmäßigerweise gemittelt werden sollte, sondern auch hinsichtlich der typischerweise zu erwartenden absoluten Dopplersignal-Zeitdauem hat sich in klinischen Experimenten eine deutliche Abhängigkeit von individuellen Patienten gezeigt. Daher kann die Dopplersignaleinheit in einer zweckmäßigen Weiterbildung des erfindungsgemäßen Lithotripters dazu ausgelegt sein, die Zeitdauer eines Dopplersignals auf die Referenz-Zeitdauer eines Referenz-Dopplersignals zu normieren. Dieses Referenz-Dopplersignal wird zweckmäßigerweise zu Beginn der Behandlung aufgenommen, wenn der Patient in der oben beschriebenen Weise derart im Lithotripter justiert worden ist, daß - wie sich anhand der bildgebenden Ortungsvorrichtung überwachen läßt - beispielsweise sein Nierenstein exakt im Stoßwellenfokus liegt. Bei der oben genannten Verlaufsanzeige, d.h. der fortlaufenden Auftragung von Signaldauern im Verlauf der Stoßwellenbehandlung, werden dann also keine absoluten Dopplersignal-Zeitdauern aufgetragen, sondern normierte Dopplersignal-Zeitdauern.

In einer Ausführungsform des erfindungsgemäßen Lithotripters kann vorgesehen sein, daß die Ultraschall-Sende/Empfangseinheit als Teil eines bildgebenden Ultraschallscanners ausgebildet ist (Duplex-Scanner). In diesem Fall können insbesondere der Ultraschall-Transducer und Teile der Elektronik der Ultraschall-Sende/Empfangseinheit gleichzeitig für die Ultraschall-Bildgebung und für die Dopplersignalmessungen eingesetzt werden.

Alternativ kann der Ultraschall-Transducer als preisgünstige Stiftsonde ausgebildet sein, was ihn bei Verwendung mit dem oben genannten Halter flexibel einsetzbar macht, um beispielsweise sicherzustellen, daß er immer auf den Stoßwellenfokus gerichtet ist.

Insbesondere empfiehlt sich eine derartige Gestaltung, wenn der erfindungsgemäße Lithotripter ferner eine Röntgenortungsvorrichtung aufweist. In diesem Fall erfolgt die Bildgebung zu Beginn der Positionierung des Patienten sowie bei der etwa alle drei bis fünf Minuten erfolgenden Detailkontrolle mit Hilfe dieser bildgebenden Röntgenortungsvorrichtung, während die kontinuierliche Trefferkontrolle mit Hilfe des als Stiftsonde gestalteten Ultraschall-Transducers erfolgt. Es versteht sich, daß auch in diesem Fall die Anzeige der Dopplersignale auf der auch für die Anzeige der Röntgenbilder eingesetzten Anzeigevorrichtung erfolgen kann.

Ein Ausführungsbeispiel der Erfindung wird im folgenden anhand der Zeichnungen erläutert werden.
Es zeigen:
- Fig. 1: eine vereinfachte Darstellung wesentlicher Bauteile des erfindungsgemäßen Lithotripters;
- Fig. 2: eine schematische Ansicht der Dopplersignaleinheit und ihrer Anschlüsse beim erfindungsgemäßen Lithotripter;
- Fig. 3: eine Darstellung von Ultraschall- und Dopplersignalen auf der Anzeigevorrichtung des erfindungsgemäßen Lithotripters;
- Fig.4: ein schematisches Beispiel für eine der weiteren Signalauswertung zugrundzulegende Einhüllende an das Dopplersignal;
- Fig. 5.: ein schematisches Flußdiagramm zur Verdeutlichung der Hauptabschnitte des erfindungsgemäßen Verfahrens;
- Fig. 6: ein Flußdiagramm zur Verdeutlichung wesentlicher Verfahrensschritte innerhalb des ersten Hauptabschnitts gemäß Fig. 5;
- Fig. 7: ein Flußdiagramm zur Verdeutlichung wesentlicher Verfahrensschritte innerhalb des zweiten Hauptabschnitts gemäß Fig. 5;
- Fig. 8: ein Flußdiagramm zur Verdeutlichung wesentlicher Verfahrensschritte des dritten Hauptabschnitts gemäß Fig. 5; und
- Fig. 9: ein schematisches Beispiel für eine Signaldauer-Verlaufskontrolle während einer Stoßwellentherapie.

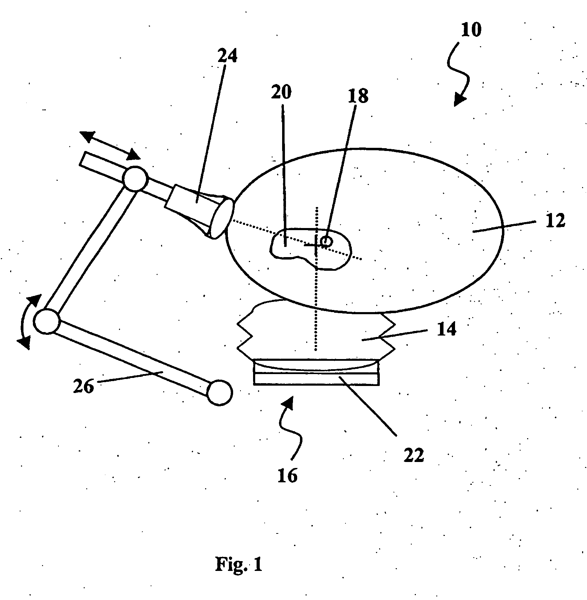

Fig. 1 zeigt eine schematische Ansicht wesentlicher mechanischer Komponenten des erfindungsgemäßen Lithotripters 10. Ein Patient 12 ist derart auf einer in Fig. 1 nicht dargestellten verstellbaren Liege gelagert, daß ein Ankoppelkissen 14 eines Stoßwellengenerators 16 an der gewünschten Stelle an den Körper des Patienten 12 gedrückt werden kann, um Stoßwellen in Richtung eines im Körper des Patienten 12 zu fragmentierenden Konkrements 18 auszusenden. In dem in Fig. 1 schematisch dargestellten Fall ist dieses Konkrement 18 ein Nierenstein in der Niere 20 des Patienten 12. Der Patient 12 wird mit Hilfe der verstellbaren Liege derart "einjustiert", d.h. positioniert, daß der in Fig. 1 durch ein Kreuz angedeutete Fokus der Stoßwellen, die von einer Stoßwellenquelle 22 des Stoßwellengenerators 16 erzeugt und mit Hilfe des Ankoppelkissens 14 in den Körper des Patienten 12 hineingesendet werden, im Nierenstein 18 liegt. Diese Justage erfolgt üblicherweise mit Hilfe einer bildgebenden Ortungsvorrichtung, beispielsweise einer Röntgenvorrichtung oder eines Ultraschallscanners.

Diese Komponenten des Lithotripters 10 und diese Vorgehensweise bei der Positionierung des Patienten vor Beginn der Behandlung sind an sich bekannt und werden hier nicht näher erläutert werden.

Der erfindungsgemäße Lithotripter 10 umfaßt ferner einen Ultraschall-Transducer 24, der an einem einstellbaren Halter 26 montiert ist. Bei dem in Fig. 1 gezeigten Beispiel ist dieser Halter 26 in Form eines gelenkigen Arms ausgebildet und ermöglicht eine genaue Positionierung des Ultraschall-Transducers 24 an einer gewünschten Stelle des Körpers des Patienten 12 derart, daß der Ultraschall-Transducer 24 - wie in Fig. 1 durch gepunktete Linien angedeutet - auf den Stoßwellenfokus gerichtet ist. Man spricht bei dieser Anordnung von einer isozentrischen Scannerführung.

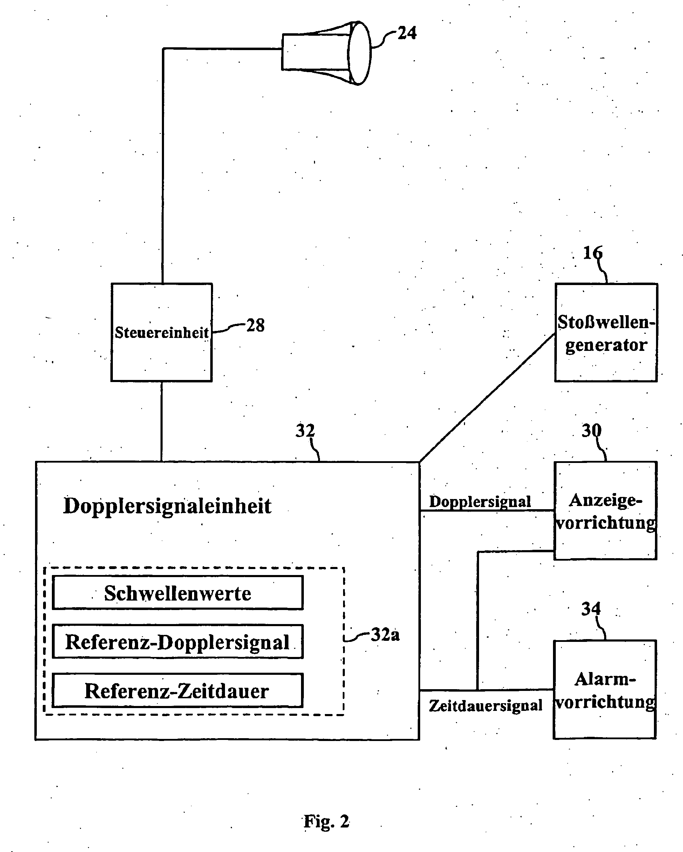

Der Ultraschall-Transducer 24 sendet kontinuierlich (continuous wave, CW) oder in Pulsen (pulsed wave, PW) Ultraschallwellen in Richtung des Stoßwellenfokus aus und empfängt ferner Ultraschallwellen, die im Körper des Patienten 12, insbesondere aus dem Gebiet des Stoßwellenfokus, reflektiert worden sind. Die empfangenen Ultraschallsignale werden, wie in Fig. 2 angedeutet, vom Ultraschall-Transducer 24 einer Steuereinheit 28 zugeführt, die nicht nur als Teil einer Sende/Empfangseinheit den eigentlichen Betrieb des Ultraschall-Transducers 24 steuert, beispielsweise die in ihm enthaltenen piezoelektrischen Elemente, und die vom Ultraschall-Transducer 24 gemessenen reflektierten Ultraschallsignale erfaßt, sondern diese auch an nachgeordnete Elektronikeinheiten weiterleitet. Beispielsweise könnte die Steuereinheit 28 die empfangenen Ultraschallsignale an ein in Fig. 2 nicht dargestelltes Bildverarbeitungsmodul weiterleiten, mit dessen Hilfe auf einer Anzeigevorrichtung 30 Ultraschallbilder des Nierensteins dargestellt werden können.

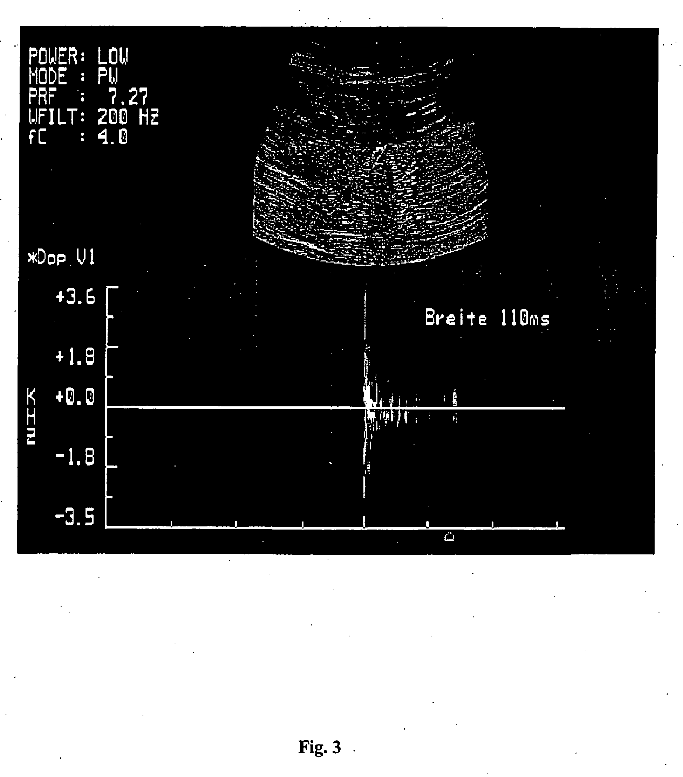

Unabhängig hiervon führt die Steuereinheit 28 beim erfindungsgemäßen Lithotripter 10 die empfangenen Ultraschallsignale einer Dopplersignaleinheit 32 zu, die basierend auf der bekannten Frequenz der vom Ultraschall-Transducer 24 ausgesandten Ultraschallwellen und den dopplerverschobenen Frequenzen der vom Ultraschall-Transducer 24 empfangenen Ultraschallwellen nach jedem Aussenden einer Stoßwelle durch den Stoßwellengenerator 16 zunächst ein Dopplersignal erzeugt. Ein solches Dopplersignal umfaßt die zeitaufgelöste Darstellung der gemessenen Frequenzverschiebungen. Ein Beispiel für ein solches von der Dopplersignaleinheit 32 erzeugtes und auf einer an die Dopplersignaleinheit 32 angeschlossenen Anzeigevorrichtung 30 dargestelltes Dopplersignal ist in Fig. 3 unten aufgetragen. Hierbei entspricht Fig. 3 einem Teil einer möglichen Anzeige des Bildschirms der Anzeigevorrichtung 30 des erfindungsgemäßen Lithotripters 10. In Fig. 3 oben erkennt man zunächst ein übliches Ultraschallbild eines Körperbereichs des Patienten 12, der das Konkrement 18 ungefähr in der Mitte enthält. In Fig. 3 unten ist das im Anschluß an eine Stoßwelle gemessene Dopplersignal in Form der gemessenen Frequenzverschiebungen (man beachte die Einheit kHz an der Ordinate des dargestellten Koordinatensystems) als Funktion der Zeit aufgetragen. Man erkennt annähernd symmetrisch zu Abszisse (Zeitachse) verteilte positive und negative Frequenzverschiebungen, deren Amplitude mit zunehmendem zeitlichen Abstand von der Aussendung der Stoßwelle kleiner wird. Ursache für dieses Signal sind die folgenden Prozesse beim Auftreffen der Stoßwelle auf das Konkrement 18:

Liegt das Konkrement 18 im Stoßwellenfokus, so daß beim Aussenden der Stoßwelle ein "Treffer" erzielt wird, so werden kleine Fragmente aus dem Konkrement 18 herausgeschlagen und gleichzeitig treten im Konkrement 18 Umschichtungen auf. In der unmittelbaren Umgebung des Konkrements 18 kommt es ferner in der körpereigenen Flüssigkeit des Patienten 12 zu erhöhter Kavitation. All diese Prozesse führen dazu, daß die vom Ultraschall-Transducer 24 in Richtung des Konkrements 18 ausgesandten Ultraschallwellen zunehmend an Objekten reflektiert werden, die gegenüber dem Transducer 24 in Bewegung sind. Da diese Bewegung ungerichtet ist, d.h. einige der aus dem Konkrement 18 herausgeschlagenen Fragmente Bewegungskomponenten auf den Ultraschall-Transducer zu aufweisen, andere Fragmente hingegen Bewegungskomponenten vom Ultraschall-Transducer 24 weg, treten hinsichtlich der Frequenzen der zum Ultraschall-Transducer 24 zurückreflektierten Schallwellen sowohl positive als auch negative Dopplerverschiebungen auf. Durch den sogenannten Spiegelartefakt werden zusätzliche - artifizielle - Anteile registriert, so daß annähernd gleich viele positive wie negative Dopplerverschiebungen auftreten. Auch die bei einem Treffer in der unmittelbaren Umgebung des Konkrements 18 verursachte erhöhte Kavitation führt zu einer ungeregelten Bewegung von reflektierenden Objekten (Dichteschwankungen bzw. Gasblasen in der Körperflüssigkeit des Patienten 12), was ebenso zu positiven und negativen Dopplerverschiebungen führt.

Auch eine makroskopische Bewegung des Konkrements 18, die insbesondere dann auftreten kann, wenn es bereits mindestens teilweise fragmentiert ist, führt zu einer zeitlichen Verteilung positiver und negativer Frequenzveränderungen, wenn das Konkrement 18 aufgrund von elastischen Kräften des umliegenden Gewebes eine Art Schwingungsbewegung vollführt.

Wie man beim Dopplersignal in Fig. 3 deutlich erkennt, sinkt sein Betrag mit zunehmendem zeitlichen Abstand von der Aussendung der Stoßwelle kontinuierlich ab, da das System zunehmend wieder zur Ruhe kommt. Nach einer gewissen charakteristischen Zeit ist keinerlei Dopplerverschiebung bei den Frequenzen der zurückreflektierten Ultraschallwellen mehr feststellbar. Es konnte festgestellt werden, daß diese Zeitdauer des Dopplersignals ein charakteristisches Maß für die Stärke der Wechselwirkung der Stoßwelle mit dem Konkrement 18 ist, und somit Informationen darüber liefern-kann, ob und in welchem Maß das Konkrement 18 getroffen wurde:
So wurde festgestellt, daß ein Treffer zu Beginn einer Lithotripsiebehandlung bei einem noch weitgehend unfragmentierten Konkrement 18 typischerweise Dopplersignale mit einer Zeitdauer von etwa 50 msec. liefert. Im Verlauf der Behandlung erhöht sich diese Zeitdauer kontinuierlich, bei einem teilweise fragmentierten Konkrement 18 beträgt sie etwa 120 msec. (man beachte die entsprechende Anzeige "Breite 110 ms" in Fig. 3 unten). Bei einem Fehlschuß hingegen tritt zwar aufgrund von schwacher Kavitation in der Körperflüssigkeit des Patienten 12 ebenfalls ein Dopplersignal auf, seine Zeitdauer beträgt jedoch typischerweise nur etwa 25 msec.

Zur Auswertung des Dopplersignals zwecks Ermittlung seiner Zeitdauer sind verschiedene Vorgehensweisen möglich, die auf dem Gebiet der Datenauswertung grundsätzlich bekannt sind:

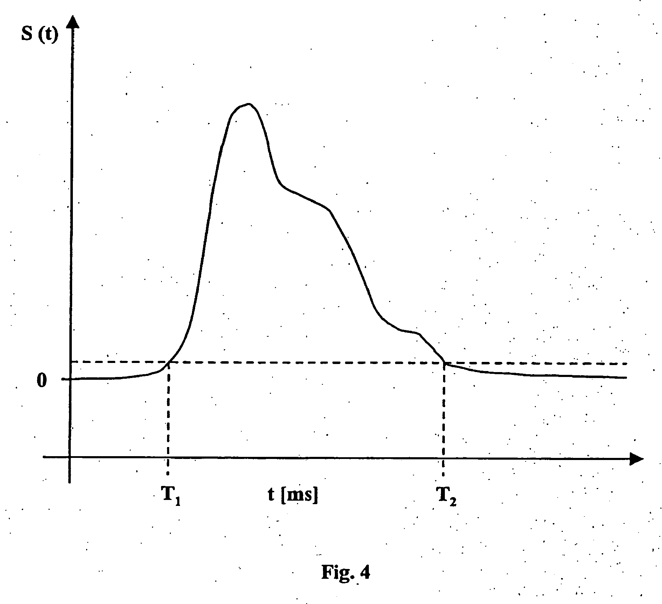

Zunächst wird das Dopplersignal in der Dopplersignaleinheit 32 zwischengespeichert und von allen gespeicherten Werten der Betrag gebildet, um nicht mit positiven und negativen Frequenzverschiebungen arbeiten zu müssen. Dann kann an die entsprechende Kurve der Betragswerte, die stark oszilliert, eine Einhüllende durch die Scheitelpunkte der kontinuierlich kleiner werdenden Halbwellen angelegt werden. Ein schematisches Beispiel für die derart ermittelte Einhüllende ist in Fig. 4 dargestellt. An diese Einhüllende läßt sich in grundsätzlich bekannter Weise beispielsweise eine fallende e-Funktion der Form exp (-t/T) anpassen (anfitten), wobei t die Zeit ab dem Aussenden der Stoßwelle bzw. ab dem Empfang der ersten zurückreflektierten Schallwelle darstellt und wobei T die gesuchte charakteristische Zeitdauer des Dopplersignals ist.

Alternativ können bei der oben bereits genannten betragsmäßigen Dopplersignalkurve ein Signal-Anfangszeitpunkt T₁ und ein Signal-Endzeitpunkt T₂ bestimmt werden, wobei T₁ dem Zeitpunkt entspricht, bei dem die beschriebene Einhüllende erstmals einen vorbestimmten ersten Schwellenwert überschreitet und T₂ dem Zeitpunkt entspricht, bei dem die Einhüllende erstmals einen zweiten Schwellenwert unterschreitet. Die gesuchte charakteristische Zeitdauer des Dopplersignals ergibt sich dann als T = T₂ - T₁. In Fig. 4 sind der erste und der zweite Schwellenwert identisch und als horizontale gestrichelte Linie angedeutet.

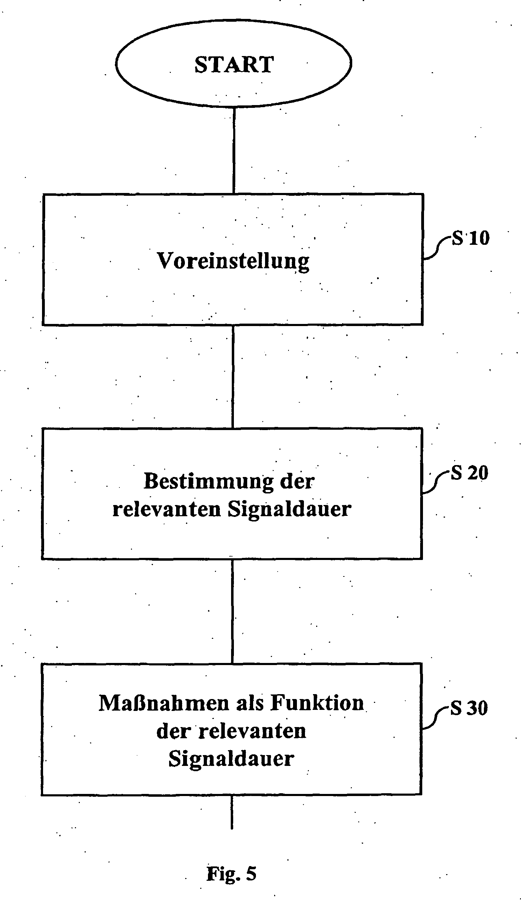

Diese Schwellenwerte werden zu Beginn der Lithotripsiebehandlung beim Aufnehmen eines Referenz-Dopplersignals ermittelt, insbesondere unter Berücksichtigung des Untergrundrauschens bei der Messung, und in einem Speicher 32a in der Dopplersignaleinheit 32 abgespeichert. Ebenso kann die dem Referenz-Dopplersignal zugeordnete Referenz-Zeitdauer im Speicher 32a im Hinblick auf eine gegebenenfalls vorzunehmende Normierung später gemessener Kurven abgespeichert werden. Dies wird weiter unten im Zusammenhang mit den Fig. 5 bis 8 erläutert werden.

Zusätzlich zu der bereits genannten Ausgabe des Dopplersignals an die Anzeigevorrichtung 30 zwecks bildlicher Darstellung entsprechend Fig. 3 unten bzw. Fig. 4 ist die Dopplersignaleinheit 32, wie in Fig. 2 gezeigt, auch mit einer Alarmvorrichtung 34 verbunden, an die sie ein zugeordnetes Zeitdauersignal ausgibt. Wie ebenfalls weiter unten erläutert werden wird, kann die Alarmvorrichtung 34 einen akustischen und/oder optischen Alarm auslösen, wenn sich aus dem Zeitdauersignal -eine zu kurze Zeitdauer des Dopplersignals ergibt, was auf einen Fehlschuß schließen läßt, d.h. darauf, daß das Konkrement 18 außerhalb des Stoßwellenfokus liegt.

Wie in Fig. 2 ferner dargestellt, ist die Dopplersignaleinheit 32 auch mit dem Stoßwellengenerator 16 verbunden, um diesen im Fall von Fehlschüssen gegebenenfalls automatisch stoppen zu können. Auf diese Weise wird eine unnötige Belastung des Körpers des Patienten 12 durch Stoßwellen-Fehlschüsse besonders sicher und wirksam vermieden, ohne daß es hierfür eines Eingreifens des medizinischen Personals bedürfte.

Wesentliche Schritte des mit dem erfindungsgemäßen Lithotripter - 10 durchführbaren erfindungsgemäßen Verfahrens werden nachfolgend anhand der Fig. 5 bis 8 erläutert werden:
Wie in Fig. 5 schematisch angedeutet, umfaßt das erfindungsgemäße Verfahren nach dem Start des erfindungsgemäßen Lithotripters im wesentlichen drei Abschnitte, nämlich einen ersten Abschnitt S10 mit Schritten zur Voreinstellung des Lithotripters vor der eigentlichen Therapie, einen anschließenden Abschnitt S20, in dem während der Therapie in verschiedenen Verfahrensschritten die relevante Dauer eines Dopplersignals bestimmt wird, sowie einen weiteren Abschnitt S30, in dem mehreren Verfahrensschritten die entsprechenden Maßnahmen als Funktion der im Abschnitt S20 ermittelten relevanten Signaldauer ergriffen werden.

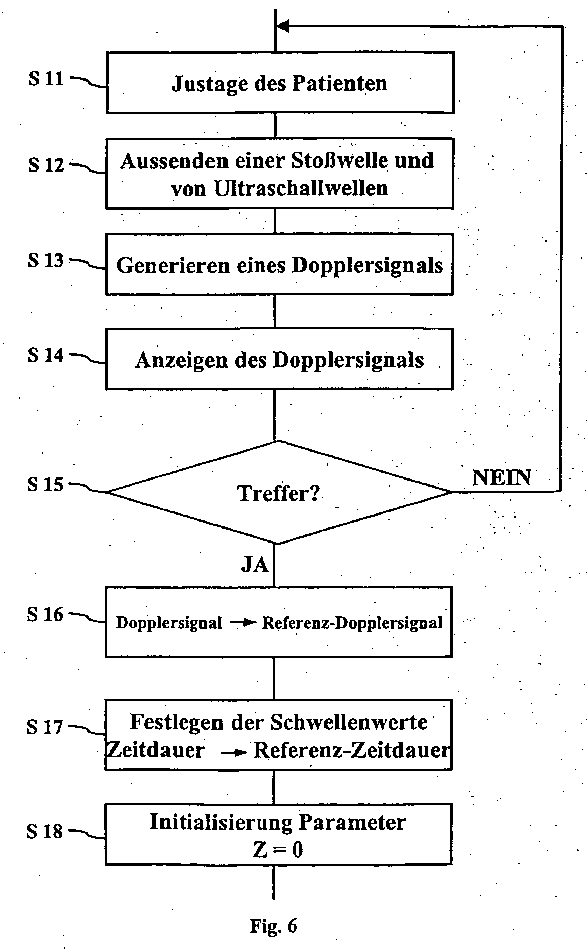

Die wichtigsten Schritte innerhalb des ersten Abschnitts S10 werden nachfolgend anhand von Fig. 6 erläutert werden:

Zunächst erfolgt im Schritt S11 die oben bereits genannte und im Stand der Technik bekannte Justage des Patienten mit Hilfe der verstellbaren Liege des Lithotripters 10. Am Ende dieser durch eine bildgebende Ortungsvorrichtung überwachten Justage ist der Patient 12 derart positioniert, daß sich sein Konkrement 18 im Stoßwellenfokus befindet.

Anschließend beginnt im Schritt S12 das Aussenden von Ultraschallwellen durch den Ultraschall-Transducer 24 sowie das erstmalige Aussenden einer Stoßwelle mit Hilfe des Stoßwellengenerators 16.

Anschließend generiert im Schritt S13 die Dopplersignaleinheit 2 anhand der reflektierten Ultraschallwellen sowie der bekannten Frequenz der vom Transducer 24 ausgesandten Ultraschallwellen ein Dopplersignal, welches anschließend im Schritt S14 auf der Anzeigevorrichtung 30 entsprechend der Fig. 3 gezeigt wird. Ebenso kann in diesem Schritt die Einhüllende an das Dopplersignal entsprechend Fig. 4 auf der Anzeigevorrichtung 30 gezeigt werden.

Dieses Dopplersignal wird im Schritt S15 vom medizinischen Personal dahingehend begutachtet, ob es auf einen Treffer schließen läßt oder auf einen Fehlschuß. Bei diesem ersten Aussenden einer Stoßwelle im Schritt S12 ergibt sich in der Regel ein Treffer, da nach dem Schritt S11 das Konkrement 18 auf jeden Fall im Stoßwellenfokus liegt, und da man vor dem Aussenden dieser einen "Test-Stoßwelle" den Patienten 12 auffordern kann, kurzzeitig die Luft anzuhalten, so daß kein Fehlschuß aufgrund der Atmungsbewegung des Konkrements droht. Bei dieser Begutachtung von Dopplersignalen dahingehend, ob ein Treffer oder ein Fehlschuß vorliegt, ist es unter anderem wichtig, das jeweilige Signalrauschen, d.h. den jeweiligen "Untergrund" bei jedem Dopplersignal, zu ermitteln.

Wird im Schritt S15 festgestellt, daß kein Treffer vorliegt, so kehrt man zu Schritt S11 zurück. Wird hingegen ein Treffer festgestellt, so wird im Schritt S16 das bereits gemessene Dopplersignal als Referenz-Dopplersignal im Speicher 32a abgespeichert. Mit Hilfe dieses Referenz-Dopplersignals werden dann im Schritt S17 die oben diskutierten Schwellenwerte festgelegt, insbesondere durch Betrachtung des Signalrauschens im Untergrund des Referenz-Dopplersignals. Ferner wird im Schritt S17 die Zeitdauer des Referenz-Dopplersignals als Referenz-Zeitdauer im Speicher 32a abgespeichert.

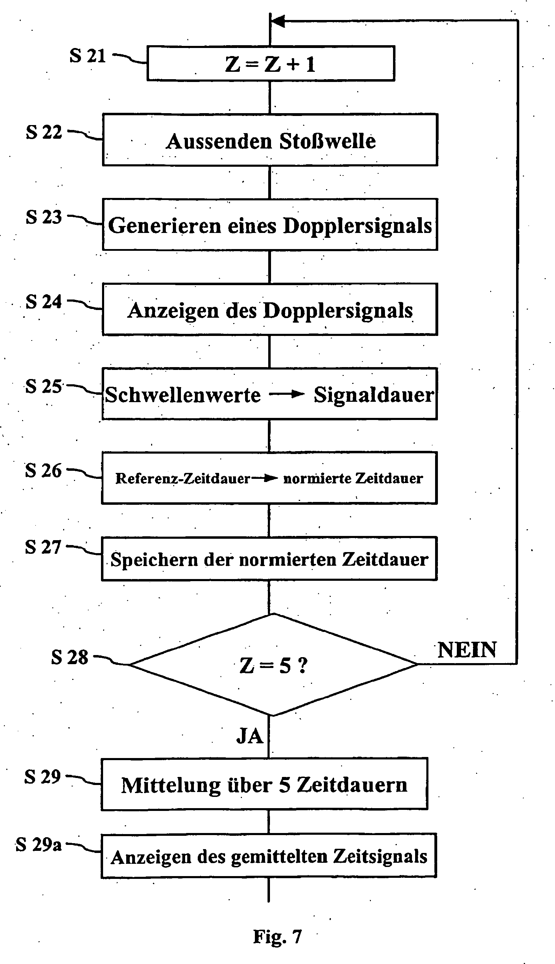

Anschließend erfolgt im Schritt S18 eine Initialisierung, d.h. ein Rücksetzen eines Zählparameters z, dessen Bedeutung nachfolgend anhand von Fig. 7 deutlich werden wird.

Fig. 7 stellt die wesentlichen Schritte innerhalb des zweiten Abschnitts S20 gemäß Fig. 5 dar, die zur Bestimmung der relevanten Signaldauer dienen.

Zunächst wird im Schritt S21 der Zählparameter z inkrementiert, d.h. um 1 erhöht. Anschließend wird im Schritt S22 eine Stoßwelle vom Stoßwellengenerator 16 ausgesandt und im Schritt S23 ein Dopplersignal von der Dopplersignaleinheit 32 generiert, welches anschließend im Schritt S24 auf der Anzeigevorrichtung 30 angezeigt wird, ebenso wie seine Einhüllende gemäß Fig. 4.

Im nachfolgenden Schritt S25 wird mit Hilfe der im Schritt S17 gemäß Fig. 6 festgelegten Schwellenwerte die Zeitdauer dieses aktuellen Dopplersignals bestimmt. Im Schritt S26 wird diese Zeitdauer mittels Division durch die ebenfalls im Schritt S17 gemäß Fig. 6 ermittelte Referenz-Zeitdauer in eine normierte Zeitdauer umgerechnet, die nachfolgend im Schritt S27 in der Dopplersignaleinheit 32 abgespeichert wird, beispielsweise in dem bereits genannten Speicher 32a oder in einem gesonderten Speicher zur Aufnahme von Meßwerten.

Dann wird im Schritt S28 überprüft, ob der Zählparameter z einen vorbestimmten Wert erreicht hat, bei dem in Fig. 7 gezeigten Beispiel den Wert z = 5. Ist dies nicht der Fall, so springt das in der Dopplersignaleinheit 32 ausgeführte Programm zum Schritt S21 zurück, in dem der Zählparameter z inkrementiert und anschließend ein weiteres Dopplersignal gemessen wird. Ergibt die Prüfung im Schritt S28 jedoch, daß der Zählparameter z den vorbestimmten Wert, hier also z = 5 erreicht hat, was bedeutet, daß die Zeitdauern von fünf zurückliegenden Dopplersignalen in der Dopplersignaleinheit 32 zwischengespeichert sind, so geht das Programm weiter zu einem Schritt S29, in dem über diese fünf zwischengespeicherten Zeitdauern gemittelt wird. Der im Schritt S29 berechnete Mittelwert der Zeitdauerwerte der letzten fünf Dopplersignale kann ebenfalls in der Dopplersignaleinheit 32 in einem der genannten Speicher zwischengespeichert werden.

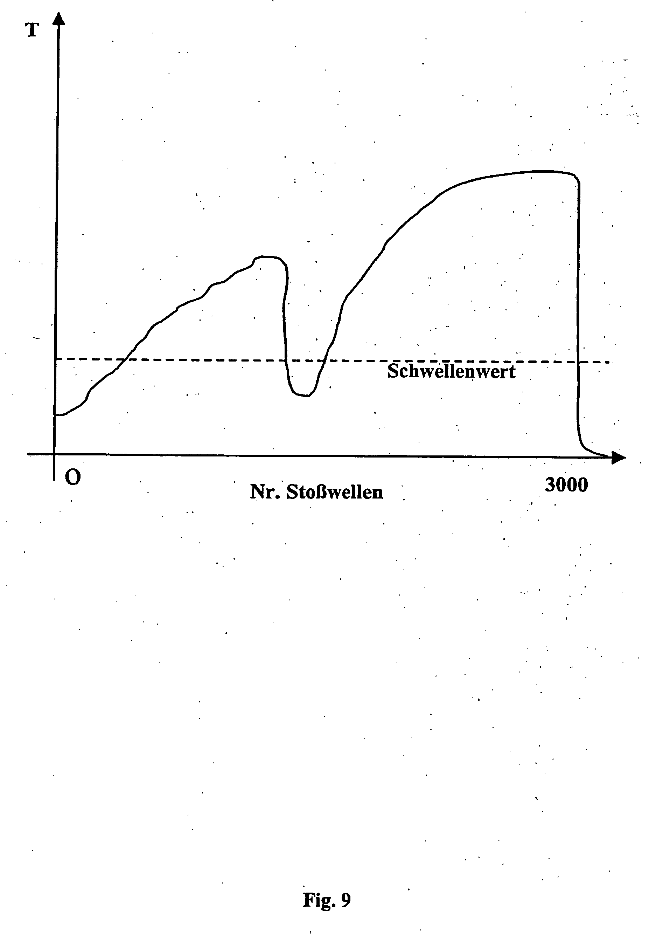

Im Schritt S29a wird der zuletzt berechnete Mittelwert einer fortlaufenden Anzeige hinzugefügt, welche die im Verlauf der gesamten Behandlung gemessenen Zeitdauern der Dopplersignale kontinuierlich darstellt, um die Entwicklung der Dopplersignallänge zu verfolgen. Zweckmäßigerweise kann diese fortlaufende Anzeige auf der Anzeigevorrichtung 30 dargestellt werden, es kann sich jedoch auch um einen gesonderten Bildschirm etc. handeln. Ein Beispiel für eine derartige fortlaufende Anzeige ist in Fig. 9 dargestellt und wird weiter unten erläutert werden.

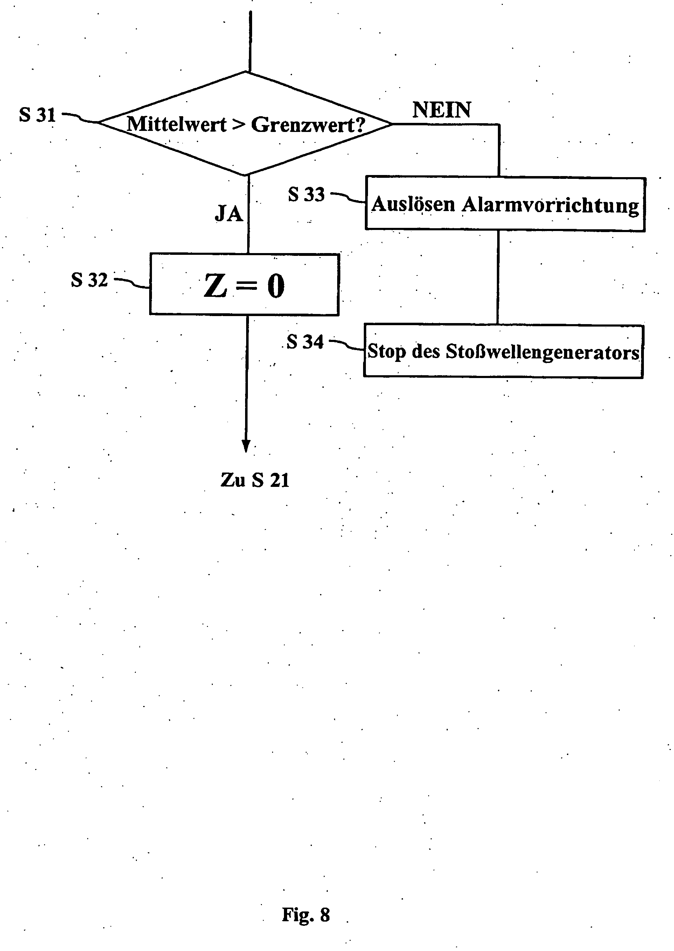

Fig. 8 zeigt die wesentlichen Schritte des dritten Abschnitts S30 gemäß Fig. 5 und die Maßnahmen, die im Rahmen des erfindungsgemäßen Verfahrens bei einer Lithotripsiebehandlung mit dem erfindungsgemäßen Lithotripter 10 als Funktion des im Schritt S29 berechneten Mittelwerts getroffen werden können:

In einem Schritt S31 wird durch das in der Dopplersignaleinheit 32 ausgeführte Programm überprüft, ob der im Schritt S29 berechnete. Mittelwert größer als ein vorbestimmter Grenzwert ist. Wie oben erläutert, deuten große Zeitdauerwerte auf Treffer hin, wohingegen kleine Zeitdauerwerte typisch für Fehlschüsse sind. Dementsprechend bedeutet ein positives Ergebnis der Prüfung im Schritt S31, daß offensichtlich das Konkrement 18 weiterhin im Stoßwellenfokus liegt und somit Treffer erfolgen. Dementspechend geht das in der Dopplersignaleinheit 32 ausgeführte Programm in diesem Fall über einen Schritt S32, in dem der Zählparameter z wiederum auf 0 zurückgesetzt wird, zurück zu Schritt S21, d.h. es beginnt erneut eine Messung von fünf normierten Dopplersignal-Zeitdauem mit anschließender Mittelung gemäß Fig. 7.

Führt die Prüfung in Schritt S31 jedoch zu einem negativen Ergebnis, d.h. dazu, daß die gemittelte Zeitdauer der letzten fünf Dopplersignale zu klein ist, was auf mögliche Fehlschüsse hinweist, so verzweigt das Programm zu einem Schritt S33, in dem die genannte Alarmvorrichtung 34 ausgelöst wird, sowie anschließend zu einem Schritt S34, in dem der Stoßwellengenerator 16 angehalten wird, um eine unnötige Belastung des Körpers des Patienten 12 durch Fehlschüsse zu vermeiden.

Fig. 9 ist eine schematische Darstellung einer beispielhaften Verlaufskontrollen-Anzeige auf der Anzeigevorrichtung 30 während einer Stoßwellentherapie. Man erkennt in Fig. 9 ein Koordinatensystem, dessen Abzisse die Zahl ausgesandter Stoßwellen und dessen Ordinate die im Anschluß an die jeweilige Stoßwelle ermittelte absolute oder normierte Doppelsignal-Zeitdauer angibt. Man erkennt vom Start der Stoßwellentherapie bis kurz vor ihrem Ende einen Verlauf entsprechend einem langsamen Anstieg der Dopplersignal-Zeitdauern. Einzelne "Einbrüche" in dieser Verlaufskurve deuten auf "Fehlschüsse" durch eine Bewegung des Patienten 12 oder des Konkrements 18 innerhalb des Patienten 12 hin, die sofort durch eine Neupositionierung des Patienten 12 korrigiert wurden. In Fig. 9 repräsentiert eine horizontale gestrichelte Linie den Betrag des Schwellenwerts, dessen Unterschreiten die Alarmvorrichtung 34 auslöst. Man erkennt, daß der lokale "Einbruch" etwa in der Mitte der Verlaufskurve zu einem Unterschreiten dieses Schwellenwerts und somit zu einem Alarm geführt hat.

Es versteht sich, daß die Fig. 5 bis 8 nur die wichtigsten Schritte des erfindungsgemäßen Verfahrens angeben, und zahlreiche vorherige, spätere oder auch eingeschobene Schritte möglich sind, die auf dem Gebiet von Lithotripsieverfahren grundsätzlich bekannt sind. Ebenso versteht es sich, daß das anhand der Fig. 5 bis 8 erläuterte Verfahren nicht zwingend eine Berechnung der Dopplersignal-Zeitdauem mit Hilfe der oben beschriebenen Schwellenwertmethode erfordert, sondern ebenso mit Hilfe der ebenfalls beschriebenen Methode des Anfittens einer e-Funktion an die Hüllkurve durchführbar ist. Ferner versteht es sich nicht nur, daß die im Schritt S28 genannte Zahl z = 5 rein beispielhaft zu verstehen ist. Insbesondere kann diese Zahl auch im Rahmen der Voreinstellungen gemäß Abschnitt S10 in Fig. 4 vom medizinischen Personal individuell eingegeben werden, beispielsweise im Schritt S17 in Fig. 6. Somit könnte beispielsweise bei einem Patienten 12 mit einem eher kleinen Konkrement 18 (das somit leichter durch die Stoßwellen zu verfehlen ist) und gleichzeitig einer schweren Atmung mit entsprechend starken Auslenkungen des Konkrements 18 über eine größere Zahl von Dopplersignal-Zeitdauem gemittelt werden als bei einem anderen Patienten 12 mit eher flacher Atmung und einem größeren Konkrement 18 (welches somit häufiger getroffen wird). Selbstverständlich kann auch ohne jede Mittelung gearbeitet werden, entsprechend z = 1.

Ebenso versteht es sich, daß die Ermittlung der Referenz-Zeitdauer (Schritt S17) und die spätere Normierung gemessener Signaldauern mit Hilfe dieser Referenz-Zeitdauer (Schritt S26) auch unterbleiben kann, so daß bei der Verlaufskontrolle während der Stoßwellentherapie Absolutwerte der gemessenen Dopplersignale-Zeitdauem der Anzeigevorrichtung 30 angezeigt und ausgewertet werden.

Hinsichtlich des anhand von Fig. 1 nur schematisch angedeuteten Lithotripters 10 versteht es sich, daß dieser zahlreiche weitere Komponenten aufweisen kann, die aus dem Stand der Technik bekannt sind, beispielsweise eine Röntgenortungsvorrichtung oder einen bildgebenden Ultraschallscanner.

### Bezugszeichenliste

- 10: Lithotripter
- 12: Patient
- 14: Ankoppelkissten
- 16: Stoßwellengenerator
- 18: Konkrement (Nierenstein)
- 20: Niere
- 22: Stoßwellenquelle
- 24: Ultraschall-Transducer
- 26: Einstellbarer Halter
- 28: Steuereinheit
- 30: Anzeigevorrichtung
- 32: Dopplersignaleinheit
- 32a: Speicher
- 34: Alarmvorrichtung

## Patentansprüche

1. Lithotripter (10) zur Fragmentierung eines Zielobjekts, insbesondere eines Konkrements (18), in einem vorzugsweise menschlichen Körper, umfassend:
- einen Stoßwellengenerator (16) zur Erzeugung fokussierter Stoßwellen,
- eine Ultraschall-Sende/Empfangseinheit mit einem Ultraschall-Transducer (24) zum Aussenden von Ultraschallwellen in den Körper und zum Empfangen von in einem Zielgebiet des Stoßwellengenerators (16) reflektierten Ultraschallwellen, und
- eine an die Ultraschall-Sende/Empfangseinheit angeschlossene Dopplersignaleinheit (32) zum Generieren und Auswerten eines Dopplersignals aus den ausgesandten und empfangenen Ultraschallwellen,
**dadurch gekennzeichnet, dass** die Dopplersignaleinheit (32) dazu ausgelegt ist, die Zeitdauer des Dopplersignals zu bestimmen und ein zugeordnetes Zeitdauersignal auszugeben.

2. Lithotripter (10) nach Anspruch 1, **dadurch gekennzeichnet, dass** er ferner eine mit der Dopplersignaleinheit (32) verbundene Anzeigevorrichtung (30) zur Anzeige von Dopplersignalen und/oder eine Alarmvorrichtung (34) aufweist, der das Zeitdauersignal zugeführt wird.

3. Lithotripter (10) nach Anspruch 1 oder 2, **dadurch gekennzeichnet, dass** der Stoßwellengenerator (16) mit der Dopplersignaleinheit (32) verbunden und dazu ausgelegt ist, die Erzeugung von Stoßwellen als Funktion des Zeitdauersignals zu stoppen bzw. fortzusetzen.

4. Lithotripter (10) nach einem der vorhergehenden Ansprüche, **dadurch gekennzeichnet, dass** der Ultraschall-Transducer (24) der Ultraschall-Sende/Empfangseinheit an einem einstellbaren Halter (26) montiert ist.

5. Lithotripter (10) nach einem der vorhergehenden Ansprüche, **dadurch gekennzeichnet, dass** die Dopplersignaleinheit (32) dazu ausgelegt ist, als Zeitdauer des Dopplersignals ein Zeitintervall zu bestimmen, innerhalb dessen der Betrag des Dopplersignals auf einen vorbestimmten Bruchteil eines Anfangsbetrags gefallen ist, insbesondere auf den e-ten Teil des Anfangsbetrags.

6. Lithotripter (10) nach einem der Ansprüche 1 bis 4, **dadurch gekennzeichnet, dass** die Dopplersignaleinheit (32) dazu ausgelegt ist, als Zeitdauer des Dopplersignals ein Zeitintervall zwischen einem Signal-Anfangszeitpunkt und einem Signal-Endzeitpunkt zu bestimmen, wobei der Signal-Anfangszeitpunkt festgestellt wird, wenn der Betrag des Dopplersignals einen ersten Schwellenwert überschreitet, und der Signal-Endzeitpunkt festgestellt wird, wenn der Betrag des Dopplersignals einen zweiten Schwellenwert unterschreitet.

7. Lithotripter (10) nach Anspruch 6, **dadurch gekennzeichnet, dass** der erste und der zweite Schwellenwert identisch sind.

8. Lithotripter (10) nach Anspruch 6 oder 7, **dadurch gekennzeichnet, dass** die Dopplersignaleinheit (32) Einstellmittel zum Einstellen des ersten und/oder des zweiten Schwellenwerts aufweist.

9. Lithotripter (10) nach einem der vorhergehenden Ansprüche, **dadurch gekennzeichnet, dass** die Dopplersignaleinheit (32) ferner dazu ausgelegt ist, die Zeitdauer des Dopplersignals über mehrere Stoßwellen zu mitteln.

10. Lithotripter (10) nach einem der vorhergehenden Ansprüche, **dadurch gekennzeichnet, dass** die Dopplersignaleinheit (32) ferner dazu ausgelegt ist, die Zeitdauer eines Dopplersignals auf die Referenz-Zeitdauer eines Referenz-Dopplersignals zu normieren.

11. Lithotripter (10) nach einem der vorhergehenden Ansprüche, **dadurch gekennzeichnet, dass** die Ultraschall-Sende/Empfangseinheit als Teil eines bildgebenden Ultraschallscanners ausgebildet ist.

12. Lithotripter (10) nach einem der vorhergehenden Ansprüche, **dadurch gekennzeichnet, dass** der Ultraschall-Transducer (24) als Stiftsonde ausgebildet ist.

13. Lithotripter (10) nach Anspruch 12, **dadurch gekennzeichnet, dass** er ferner eine Röntgenortungsvorrichtung aufweist.

14. Lithotripter (10) nach einem der vorhergehenden Ansprüche, **dadurch gekennzeichnet, dass** er Mittel zur fortlaufenden Darstellung der Zeitdauer der Dopplersignale über den Behandlungsverlaufumfasst.

## Claims

1. Lithotripter (10) for fragmenting a target object, in particular a concrement (18), in a preferably human body, comprising:
- a shock-wave generator (16) for generating focused shock waves,
- an ultrasonic transceiver with an ultrasonic transducer (24) for transmitting ultrasonic waves into the body and for receiving ultrasonic waves reflected in a target area of the shock-wave generator (16), and
- a Doppler-signal unit (32), attached to the ultrasonic transceiver, for generating and evaluating a Doppler signal from the transmitted and received ultrasonic waves,
**characterised in that** the Doppler-signal unit (32) is designed to determine the duration of the Doppler signal and to emit an assigned duration signal.

2. Lithotripter (10) according to Claim 1, **characterised in that** it further exhibits a display device (30), connected to the Doppler-signal unit (32), for displaying Doppler signals, and/or an alarm device (34) to which the duration signal is supplied.

3. Lithotripter (10) according to Claim 1 or 2, **characterised in that** the shock-wave generator (16) is connected to the Doppler-signal unit (32) and is designed to stop or to continue the generation of shock waves as a function of the duration signal.

4. Lithotripter (10) according to one of the preceding claims, **characterised in that** the ultrasonic transducer (24) of the ultrasonic transceiver is mounted on an adjustable holder (26).

5. Lithotripter (10) according to one of the preceding claims, **characterised in that** the Doppler-signal unit (32) is designed to determine, by way of duration of the Doppler signal, a time-interval within which the magnitude of the Doppler signal has fallen to a predetermined fraction of an initial magnitude, in particular to the e-th part of the initial magnitude.

6. Lithotripter (10) according to one of Claims 1 to 4, **characterised in that** the Doppler-signal unit (32) is designed to determine, by way of duration of the Doppler signal, a time-interval between a signal start-time and a signal end-time, the signal start-time being established when the magnitude of the Doppler signal exceeds a first threshold value, and the signal end-time being established when the magnitude of the Doppler signal falls below a second threshold value.

7. Lithotripter (10) according to Claim 6, **characterised in that** the first and second threshold values are identical.

8. Lithotripter (10) according to Claim 6 or 7, **characterised in that** the Doppler-signal unit (32) exhibits adjusting means for adjusting the first and/or second threshold value.

9. Lithotripter (10) according to one of the preceding claims, **characterised in that** the Doppler-signal unit (32) is further designed to average the duration of the Doppler signal over several shock waves.

10. Lithotripter (10) according to one of the preceding claims, **characterised in that** the Doppler-signal unit (32) is further designed to normalise the duration of a Doppler signal to the reference duration of a reference Doppler signal.

11. Lithotripter (10) according to one of the preceding claims, **characterised in that** the ultrasonic transceiver takes the form of part of an imaging ultrasonic scanner.

12. Lithotripter (10) according to one of the preceding claims, **characterised in that** the ultrasonic transducer (24) takes the form of a pen-type probe.

13. Lithotripter (10) according to Claim 12, **characterised in that** it further exhibits an X-ray locating device.

14. Lithotripter (10) according to one of the preceding claims, **characterised in that** it includes means for continuous representation of the duration of the Doppler signals over the course of treatment.

## Revendications

1. Lithotripteur (10) pour la fragmentation d'un objet cible, en particulier un calcul (18), dans un corps, de préférence humain, comprenant :
- un générateur d'ondes de choc (16) pour la génération d'ondes de choc focalisées,
- une unité d'émission/de réception d'ultrasons avec un transducteur d'ultrasons (24) pour l'émission d'ondes ultrasonores dans le corps et pour la réception d'ondes ultrasonores réfléchies dans une zone cible du générateur d'ondes de choc (16), et
- une unité de signal doppler (32) raccordée à l'unité d'émission/de réception d'ultrasons pour la génération et l'exploitation d'un signal doppler à partir des ondes ultrasonores émises et reçues,
**caractérisé en ce que** l'unité de signal doppler (32) est conçue pour déterminer la durée du signal doppler et pour émettre un signal de durée associé.

2. Lithotripteur (10) selon la revendication 1, **caractérisé en ce qu'**il comprend, en outre, un dispositif d'affichage (30) raccordé à l'unité de signal doppler (32) pour l'affichage de signaux doppler et/ou un dispositif d'alarme (34) auquel le signal de durée est envoyé.

3. Lithotripteur (10) selon la revendication 1 ou 2, **caractérisé en ce que** le générateur d'ondes de choc (16) est raccordé à l'unité de signal doppler (32) et est conçu pour arrêter ou poursuivre la génération d'ondes de choc en fonction du signal de durée.

4. Lithotripteur (10) selon l'une quelconque des revendications précédentes, **caractérisé en ce que** le transducteur d'ultrasons (24) de l'unité d'émission/de réception d'ultrasons est monté sur un support réglable (26).

5. Lithotripteur (10) selon l'une quelconque des revendications précédentes, **caractérisé en ce que** l'unité de signal doppler (32) est conçue pour déterminer comme durée du signal doppler un intervalle de temps dans les limites duquel la valeur du signal doppler a diminué jusqu'à une fraction prédéterminée d'une valeur de départ, en particulier jusqu'à la énième partie de la valeur de départ.

6. Lithotripteur (10) selon l'une quelconque des revendications 1 à 4, **caractérisé en ce que** l'unité de signal doppler (32) est conçue pour déterminer comme durée du signal doppler un intervalle de temps entre un point de départ de signal et un point final de signal, le point de départ de signal étant fixé lorsque la valeur du signal doppler dépasse une première valeur seuil et le point final de signal étant fixé lorsque la valeur du signal doppler est inférieure à une deuxième valeur seuil.

7. Lithotripteur (10) selon la revendication 6, **caractérisé en ce que** la première et la deuxième valeur seuil sont identiques.

8. Lithotripteur (10) selon la revendication 6 ou 7, **caractérisé en ce que** l'unité de signal doppler (32) comporte des moyens de réglage pour le réglage de la première et/ou de la deuxième valeur seuil.

9. Lithotripteur (10) selon l'une quelconque des revendications précédentes, **caractérisé en ce que** l'unité de signal doppler (32) est conçue, en outre, pour calculer la moyenne de la durée du signal doppler sur plusieurs ondes de choc.

10. Lithotripteur (10) selon l'une quelconque des revendications précédentes, **caractérisé en ce que** l'unité de signal doppler (32) est conçue, en outre, pour normer la durée d'un signal doppler à la durée de référence d'un signal doppler de référence.

11. Lithotripteur (10) selon l'une quelconque des revendications précédentes, **caractérisé en ce que** l'unité de d'émission/de réception d'ultrasons est conçue comme un composant d'un scanner d'imagerie ultrasonore.

12. Lithotripteur (10) selon l'une quelconque des revendications précédentes, **caractérisé en ce que** le transducteur d'ultrasons (24) est conçu comme une sonde à crayon optique.

13. Lithotripteur (10) selon la revendication 12, **caractérisé en ce qu'**il comprend, en outre, un détecteur de position à rayons X.

14. Lithotripteur (10) selon l'une quelconque des revendications précédentes, **caractérisé en ce qu'**il comprend des moyens pour la représentation continue de la durée des signaux doppler pendant le déroulement du traitement.
